# EUROPEAN PATENT APPLICATION

(11) **EP 3 881 858 A1**
(43) Date of publication of application: **22.09.2021**
(21) Application number: 20164446.5
(22) Date of filing: 20.03.2020
(51) Int. Cl.: A61K 38/18, A61P 19/00, A61P 21/00, A61P 19/02, A61K 35/19, A61K 31/728, C12N 5/078

(54) **HEAT-TREATED PLATELET-DERIVED GROWTH FACTOR EXTRACT FOR USE IN A METHOD OF PREVENTING OR TREATING A TISSUE DEFECT**

(71) Applicant: Pangersic, Rok, 9242 Krizevci pri Ljutomeru (SI)
(72) Inventor: Pangersic, Rok, 9242 Krizevci pri Ljutomeru (SI)
(74) Representative: Engelhard, Markus

(57) **Abstract**

The present invention relates to a platelet-derived growth factor extract for use in a method of preventing or treating a tissue defect, wherein said platelet-derived growth factor extract is heat-treated. The present invention further relates to a pharmaceutical preparation comprising a platelet-derived growth factor extract. Furthermore, the present invention relates to a method of preparing a platelet-derived growth factor extract.

## Description

### FIELD OF THE INVENTION

The present invention relates to a platelet-derived growth factor extract for use in a method of preventing or treating a tissue defect, wherein said platelet-derived growth factor extract is heat-treated. The present invention further relates to a pharmaceutical preparation comprising a platelet-derived growth factor extract. Furthermore, the present invention relates to a method of preparing a platelet-derived growth factor extract.

### BACKGROUND OF THE INVENTION

Musculoskeletal (MSK) disorders are the second most common cause of disability worldwide, measured by years lived with disability (YLDs), according to Global Burden of Disease (GBD) study. The prevalence of disabilities due to MSK disorders such as osteoarthritis has increased about 45 % from 1990 to 2010, and is expected to continue to increase due to an increasingly obese, sedentary, and ageing population. The most common MSK disorders are low back pain and neck pain, which are still poorly understood conditions. In the context of the biopsychosocial model, standard treatment has been performed for the past decades, and the clinical guidelines promote an approach in which 85-90 % of the patients do not receive a pathoanatomical diagnosis. Clinical practitioners are often left with a trial and error strategy with regard to diagnosis and treatment, and typically apply generic symptomatic treatments, such as the advice to exercise, the prescription of analgesic medications, and the provision of reassurance. However, systematic reviews reveal that existing treatments only have a minor effect, at best, regardless of whether interventions are based on biological, psychological, or social approaches. [1]

Osteoarthritis (OA) is the most common joint disorder, causing pain and functional disability. The prevalence of OA is anticipated to further increase due to an increasingly obese and ageing population. OA is associated with low-grade inflammation, but the underlying pathogenic mechanisms are not fully understood. Currently, there is no curative treatment for OA. [1,2]

Lameness is an abnormal gait or stance of an animal that is the result of dysfunction of the locomotor system. In horses, it is most commonly caused by pain, in some cases also due to neurologic or mechanical dysfunction. Lameness is a common problem in veterinary medicine, for example, lameness of racehorses, sport horses, or pleasure horses. It is regarded as one of the costliest health issues in the equine industry, both monetarily due to the costs of diagnosis and treatment, and due to the time off for healing resulting in loss of use.

A recent study has suggested that, in the US, every one in four dogs suffers from osteoarthritis and related diseases [3]. In the EU, about 10 to 20 million dogs are expected to suffer from musculoskeletal conditions. Horses are even more prone to OA; OA impacts 60 % of all horses. In the EU, about 4 million horses are expected to suffer from musculoskeletal diseases. There are various factors influencing onset of the disease and prevalence among different groups of animals.

There have been attempts to treat musculoskeletal cells and/or defects with blood preparations.

U.S. Patent No. 9688959 discloses a platelet lysate which is depleted of fibrinogen as well as a use thereof as a substitute for xenogeneic foetal bovine serum in cell culturing.

Platelet-rich plasma (PRP) has been used in therapeutic applications [4].

However, allogeneic platelet-rich plasma therapy presents risks with regard to the different blood types and potential antigens that may cause inflammatory responses. Evidence suggests that PRP therapy can exhibit pro-inflammatory activity causing ineffectiveness of PRP therapy in certain conditions [5].

Anitua et al. [6] performed a heat inactivation at 56°C for 1 h on autologous cytokine-rich plasma eye-drops. However, autologous formulations are disadvantageous with respect to providing therapeuticals which are scalable, rather standardized, and obtained from a healthy donor.

Klatte-Schultz et al. [7] demonstrated that a treatment of tendons using a platelet lysate, such as a pooled allogeneic platelet lysate, resulted in inflammation and poorer clinical outcomes.

Wang et al. [8] demonstrated, that the complement system plays a central role in osteoarthritis development.

Accordingly, the present invention aims at providing a blood preparation, such as an allogeneic blood preparation, for use in the prevention or treatment of tissue defects which has reduced immunogenicity. Furthermore, the present invention aims at providing a platelet-derived growth factor extract for use in a method of preventing or treating a tissue defect, preferably a musculoskeletal tissue defect, which has a therapeutic effect on said tissue, as well as a method or providing such an extract. The present invention further aims at providing a method of preparing an blood extract having reduced immunogenicity which is suitable to produce said extract in higher quantities since pooled allogeneic blood samples may be used, thus providing a method for industrial-scale manufacturing of allogeneic platelet-derived growth factor extracts.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a platelet-derived growth factor extract for use in a method of preventing or treating a tissue defect, wherein said platelet-derived growth factor extract is a heat-treated platelet-derived growth factor extract.

In one embodiment, in said platelet-derived growth factor extract for use, due to said heat-treatment of said platelet-derived growth factor extract, immune-activating molecules, preferably molecules of the complement system, have been inactivated.

In one embodiment, said tissue defect is a tissue inflammation, and/or is a tissue lesion deriving from a tissue injury and/or from a surgical procedure such as an organ transplantation.

In one embodiment, said tissue defect is a defect of a musculoskeletal tissue such as a tendon, a ligament, a cartilage, a muscle, or a bone, or is a defect of a cutaneous or subcutaneous tissue.

In one embodiment, said tissue defect is a medical condition selected from degenerative arthritis, a cartilage injury, a wound such as a dermal and transdermal wound, a muscle injury, and a tendinopathy such as a tendon injury or tendinitis.

In one embodiment, said method of preventing or treating a tissue defect is a method of promoting wound healing.

In one embodiment, said extract is prepared from blood of an animal, preferably a mammal, more preferably a horse, a dog, a cat, a cow, a pig, a sheep, a monkey, or a human.

In one embodiment, said method of preventing or treating comprises administering said extract to a patient in need thereof, wherein said extract is prepared from autologous and/or allogeneic blood.

In one embodiment, said extract is formulated as an allogeneic formulation.

In one embodiment, in said method, said extract is administered locally, topically, and/or systemically.

In one embodiment, in said method, said extract is administered intratendinously, intramuscularly, intraligamentarily, intraosseously, intragastrointestinally, intraperitoneally, orally, nasally, mucosally, intrabronchially, intrapulmonarily, intradermally, intracoronarily, subcutaneously, intravenously, intravascularly, intrathecally, intraocularly, intraarticularly, or intranodally.

In one embodiment, said heat-treatment comprises, preferably consists of, a heat-treatment of said extract at a temperature of from 50 °C to 70 °C for a period of 15 min to 60 min, preferably at a temperature of from 55 °C to 60 °C for 30 min to 60 min, more preferably at a temperature of 56 °C for 30 min.

In one embodiment, said heat-treatment of said platelet-derived growth factor extract reduces immunogenic activity of said platelet-derived growth factor extract.

In one embodiment, said extract comprises any of platelet-derived growth factor, transforming growth factor, platelet factor interleukin, platelet-derived angiogenesis factor, vascular endothelial growth factor, epidermal growth factor, insulin-like growth factor, and combinations of the foregoing.

In one embodiment, in said method, said extract is co-administered with a therapeutic agent.

In one embodiment, in said method, said extract is prepared by providing a blood sample comprising platelets and extracting growth factors from said platelets.

In a further aspect, the present invention relates to a pharmaceutical preparation comprising the platelet-derived growth factor extract as defined above.

In one embodiment, said preparation further comprises any of fibronectin, collagen, hyaluronic acid, cord blood lysate, and an excipient.

In one embodiment, said preparation further comprises any of fibronectin nanofibers, a collagen type 1 and/or 2 gel, hyaluronic acid, such as hyaluronic acid 1,0 mg/mL, cord blood lysate (such as 1% cord blood lysate), and an excipient.

In one embodiment, said preparation is a preparation for use in a method of preventing or treating a tissue defect.

In this aspect, said platelet-derived growth factor extract, said use, and said method of preventing or treating a tissue defect are as defined above.

In a further aspect, the present invention relates to a method of preparing a heat-treated platelet-derived growth factor extract as defined above, comprising the following steps:
a) providing a blood sample comprising platelets, and preparing a red blood cell fraction and a plasma fraction comprising platelets from said blood sample,
b) homogenizing said plasma fraction comprising platelets; optionally followed by obtaining a platelet-poor plasma supernatant and a platelet-rich pellet, and resuspending said platelet-rich pellet, preferably in a fraction of said platelet-poor plasma supernatant,
c) releasing cytokines and growth factors from said platelets in the preparation obtained in step b),
d) deactivating immune-activating molecules by heat-treatment of the growth factor extract obtained in step c), and
e) obtaining heat-treated platelet-derived growth factor extract, optionally comprising sterile filtration with optional prefiltration of said heat-treated platelet-derived growth factor extract.

In one embodiment, said method of preparing a heat-treated platelet-derived growth factor extract comprises a step of cooling an extract obtained in step d) after heat-treatment, wherein said step of cooling is preferably performed between steps d) and e), and/or is performed at the beginning of step e).

In one embodiment, said blood sample is an autologous blood sample and/or an allogeneic blood sample, and wherein, optionally, said blood sample is a pooled blood sample.

In one embodiment, said releasing comprises any of shock-freezing, treatment with CaCl2, and ultrasound treatment of said platelets, preferably comprises shock-freezing followed by ultrasound treatment of said platelets.

In one embodiment, said deactivating comprises, preferably consists of, a heat-treatment at a temperature of from 50 °C to 70 °C for a period of 15 min to 60 min, preferably at a temperature of from 55 °C to 60 °C for 30 min to 60 min, more preferably at a temperature of 56 °C for 30 min.

In this aspect, said platelet-derived growth factor extract, said blood, said heat-treatment, and said method of preventing or treating a tissue defect are as defined above.

In a further aspect, the present invention relates to a method of preventing or treating a tissue defect, comprising administering to a patient in need thereof an effective amount of a heat-treated platelet-derived growth factor extract or a pharmaceutical preparation comprising a heat-treated platelet-derived growth factor extract.

In this aspect, said tissue defect, said platelet-derived growth factor extract, said administering, said patient, and said pharmaceutical preparation are as defined above.

In a further aspect, the present invention relates to a use of a heat-treated platelet-derived growth factor extract or a pharmaceutical preparation comprising a heat-treated platelet-derived growth factor extract for the manufacture of a medicament for a method of preventing or treating a tissue defect.

In this aspect, said platelet-derived growth factor extract, said pharmaceutical preparation, and said tissue defect are as defined above.

### DETAILED DESCRIPTION

The present invention relates to a heat-treated platelet-derived growth factor extract for use in a method of preventing or treating a tissue defect such as a skeletal or soft tissue lesion, a degenerative or non-degenerative joint condition, a tendon injury, a chronic tendonitis, a wound, a bone or muscle tissue injury, a connective tissue incision, or a dermal or subdermal defect of the skin of a mammal. Furthermore, the present invention relates to a growth factor and cytokine concentrated plasma, such as an allogeneic pooled growth factor and cytokine concentrated plasma (APGFCCP), optionally in combination with a suspension of fibronectin nanofibers in hyaluronic acid or fibronectin-fibrin gel. A platelet-derived growth factor extract for use according to the present invention and a pharmaceutical preparation according to the present invention are therapeutically useful in the human and veterinary medicine.

A platelet-derived growth factor extract for use according to the present invention can be manufactured in large scale, thus enabling a new way of quality standard treatment of multiple diseases and conditions such as musculoskeletal tissue defects.

The term "platelet-derived growth factor extract", as used herein, relates to a preparation of blood. In many of the embodiments, the term "platelet-derived growth factor extract" relates to a heat-treated platelet-derived growth factor extract. In one embodiment, said extract is useful in promoting the healing of a wound. In one embodiment, said extract is enriched in growth factors and/or cytokines compared to whole blood. In one embodiment, said extract may relate to an Allogeneic Growth Factor and Cytokine Concentrated Plasma (AGFCCP), an Allogeneic Pooled Growth Factor and Cytokine Concentrated Plasma (APGFCCP), Platelet rich growth factor concentrate (PRGFC), or Platelet Concentrated Plasma (PCP). In one embodiment, a platelet-derived growth factor extract is heat-treated to deactivate immune-activating molecules, i.e. to reduce the immunogenicity of the extract. In one embodiment, an extract of the present invention is preferably an allogeneic pooled heat-treated growth factor and cytokine rich concentrated plasma. In one embodiment, the terms "growth factor and cytokine rich concentrated plasma" and "platelet-derived growth factor extract" are used synonymously. In one embodiment, the terms "platelet-derived growth factor extract", "Allogeneic Pooled Growth Factor and Cytokine Concentrated Plasma", and "Allogeneic Growth Factor and Cytokine Concentrated Plasma" are used interchangeably. In one embodiment, when referring to an "extract", a platelet-derived growth factor extract is meant, preferably a heat-treated platelet-derived growth factor extract is meant. In one embodiment, a platelet-derived growth factor extract is APGFCCP, and is optionally combined with cord blood platelet lysate or hyaluronic acid. In one embodiment, a cord blood lysate is dispensed in heat-treated extract, such as cord blood lysate is dispensed in a concentration of 1% in heat-treated extract. In one embodiment, a platelet-derived growth factor extract is combined with fibronectin nanofibers which are prepared in accordance with [9]. In one embodiment, an extract is obtained by i) lysis, such as via freezing and then thawing, via activation through addition of CaCl2, or via ultrasound, resulting in platelets breaking apart and releasing growth factors, ii) removal of debris through centrifugation and/or filtration, iii) heat-treatment resulting in coagulation and/or removal of complement, iv) cooling to about 4 -7°C and optionally subsequent removal of coagulated content using filtration. In one embodiment, cooling the extract to about 4 -7°C after heat-treatment is very important to maintain the healing-enabling properties of the extract. In one embodiment, a platelet-derived growth factor extract of the present invention comprises enhanced levels of cytokines promoting wound healing compared to allogeneic plasma, such as angiostatin, PDGF-AB, and IGFBP-3. In one embodiment, a platelet-derived growth factor extract is different from allogeneic plasma in markers OPN, Angiostatin, IL-23, PDGF-AB, sgp130, Siglec-5, GDF-15, IGFBP-3, MCSFR, VEGF R2, Eotaxin, Eotaxin-2, RAGE, IL-6 sR, IL-16, and MIP-ib. In one embodiment, a platelet-derived growth factor extract is different from allogeneic platelet lysate in markers OPN, angiostatin, IL-23, PDGF-AB, sgp130, Siglec-5, GDF-15, IGFBP-3, MCSFR, VEGF R2, Eotaxin, Eotaxin-2, RAGE, IL-6 sR, IL-16, MIP-1b, EG-VEGF, FGF-7, SCF R, IL-6, and VEGF-C. In one embodiment, angiostatin is an angiogenesis inhibitor. In one embodiment, IL-23 is a proinflammatory cytokine having a role in Th17 maintenance and expansion, and promoting angiogenesis. In one embodiment, PDGF-AB is a mitogenic growth factor having a potent effect on mesenchymal cell lines and promoting wound healing. In one embodiment, sgp130 is an IL-6 inhibitor on trans-signaling which traps free IL-6. In one embodiment, Siglec-5 is sialic acid-binding Ig-like lectin 5. In one embodiment, GDF-15 regulates inflammatory pathways. In one embodiment, IGFBP-3 is a main IGF transportation protein, interacting with cell-surface proteins and having pro- and anti-proliferative effects on different cell types. In one embodiment, M-CSF R is a receptor for M-CSF which is released by osteoblasts and exerts paracrine effects on osteoclasts. In one embodiment, VEGF 2R is a high affinity receptor for VEGF-A expressed in many vascular endothelial cells. In one embodiment, Eotaxin is an eosinophil chemotactic protein selectively recruiting eosinophils, showing increased levels in plasma associated with aging in humans and mice. In one embodiment, Eotaxin-2 is strongly chemotactic for resting T lymphocytes and slightly chemotactic for neutrophils. In one embodiment, IL-6 sR is elevated, together with IL-6 and sgp130, in chronic spontaneous urticarial, and triggers osteoclast formation in the presence of IL-6. In one embodiment, MIP-1b is a chemotactic cytokine expressed in multiple cell lines and platelets upon activation, and induces the synthesis and release of pro-inflammatory cytokines. In one embodiment, RAGE is a pattern recognition immunoglobulin-like receptor, existing in membrane-bound (mRAGE) and soluble form (sRAGE), that induces inflammation.

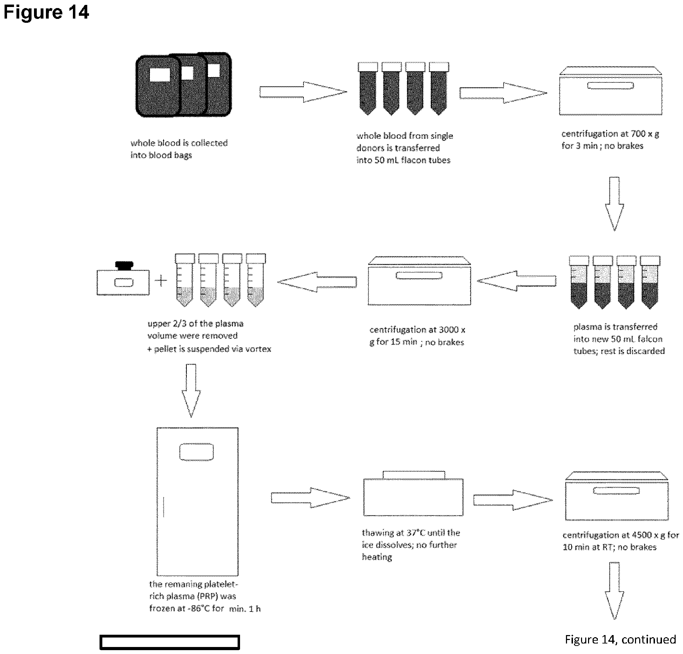

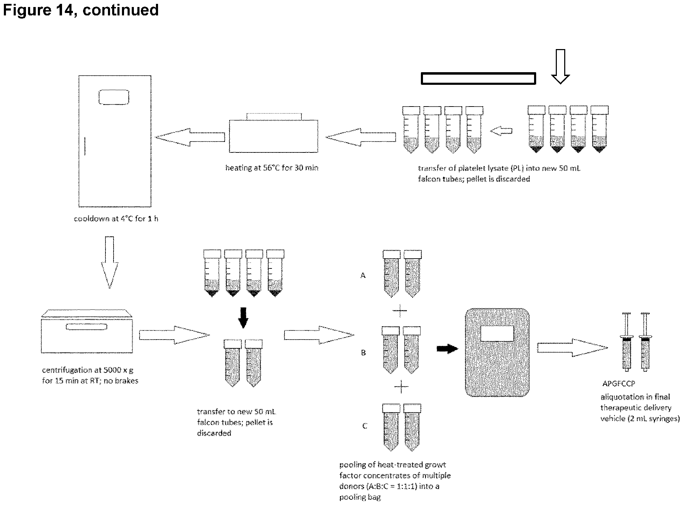

In one embodiment, obtaining a platelet-derived growth factor extract comprises obtaining PRP using a PRP method and/or a buffy coat method. In one embodiment obtaining a platelet-derived growth factor extract comprises any, preferably all of the following steps: i) obtaining whole blood, such as autologous blood or allogeneic blood from one or more donors, ii) centrifuging whole blood at a speed of about 400 to 1000 x g, preferably about 700 x g, for about 0.5 to 10 min, preferably for about 3 min, iii) transferring thereby obtained plasma into new tubes, iv) centrifuging said plasma at a speed of about 1000 to 5000 x g, preferably about 3000 x g, for about 1 to 30 min, preferably for about 15 min, v) removing the upper part of the plasma, e.g. the upper 2/3 of the plasma, vi) suspending the pellet, such as via vortexing, vii) freezing the remaining PRP, such as at about - 80 °C for about 1 min, viii) thawing the sample, such as at 37 °C, until the ice has dissolved, ix) centrifuging said sample at a speed of about 1000 to 7000 x g, preferably about 4500 x g, for about 1 to 30 min, preferably for about 10 min, x) transferring thereby obtained platelet lysate into new tubes and heat treating the platelet lysate, such as at a temperature of about 40 °C to 65 °C, preferably about 56 °C, for 1 to 60 min, preferably about 30 min, xi) cooling down to a temperature of about 4 °C to 8 °C, for 10 min to 2 h, such as 1 h, xii) centrifuging the sample at a speed of about 1000 to 7000 x g, preferably about 5000 x g, for about 1 to 30 min, preferably for about 15 min, xiii) obtaining the supernatant which is a heat-treated extract of the present invention, optionally pooling supernatants of different samples to obtain a pooled supernatant, and optionally storing said supernatant for further use, such as for use in treating a tissue defect. In one embodiment, a heat-treated extract of the present invention is an extract obtained using a method comprising steps i)-xiii). In one embodiment, an extract of the present invention is an extract obtained using a method as depicted schematically in Figure 14. In one embodiment, said storing comprises using the obtained extract freshly, i.e. directly after preparation, or freezing said extract, such as for up to one month at -20 °C and/or for one month or more than one month at a temperature of about - 80 °C to - 90 °C. In one embodiment, an extract of the present invention is obtained by using a PRP method to obtain PRP, subsequently lysing the platelets in the PRP to obtain a platelet lysate, and heat-treating the platelet lysate. In one embodiment, a heat-treated extract is superior compared to a non-heat-treated extract in that it is less immunogenic. In one embodiment, a heat-treated extract of the present invention comprises cytokines and/or growth factors that stimulate wound healing.

The term "tissue defect", as used herein, relates to any defect of a tissue such as a musculoskeletal tissue. It may relate to, for example, a tissue inflammation or a tissue lesion deriving from a tissue injury and/or from a surgical procedure such as an organ transplantation. In one embodiment, a tissue defect relates to a defect such as a skeletal or soft tissue lesion, a degenerative or non-degenerative joint condition, a tendon injury, a chronic tendonitis, a wound, a bone or muscle tissue injury, a connective tissue incision, or a dermal or subdermal defect of the skin of a mammal. In a preferred embodiment, a tissue defect is a soft-tissue defect, preferably a skin laceration, a tendinopathy such as tendinitis, arthritis such as arthritis caused by aseptic inflammation or degeneration, or a tendon injury. In an alternative embodiment, the defect may further be a defect of bone tissue and/or muscular tissue. In one embodiment, the tissue defect relates to an aging-associated tissue defect, such as a wrinkle. In one embodiment, the platelet-derived growth factor extract of the present invention is for use in aesthetic applications, such as for use as a filler material in treating and/or preventing wrinkles.

The term "heat-treated", as used herein, relates to heating of an blood extract for a defined period of time at a defined temperature range for reducing the immunogenic potential of the blood extract. In one embodiment, said heat-treating refers to a heating of said extract at a temperature of from 50 °C to 70 °C for a period of 15 min to 60 min, preferably at a temperature of from 55 °C to 60 °C for 30 min to 60 min, more preferably at a temperature of 56 °C for 30 min. In one embodiment, heat-treatment relates to treating an extract at a temperature of from 50 °C to 60 °C for 15 min to 60 min, more preferably at a temperature of 56 °C for 30 min. In one embodiment, "heat-treated" refers to a heat-treatment of an extract prior to use of said extract in a method of preventing or treating a tissue defect. In one embodiment, a heat-treated platelet-derived growth factor extract for use in a method of preventing or treating a tissue defect is an extract which has been subjected to a heat-treatment prior to its use in a method of preventing or treating a tissue defect. In one embodiment, said heat-treatment inactivates immune-activating molecules such as complement and reduces immunogenicity of said extract.

The term "immune-activating molecules", as used herein, relates to molecules that activate the immune system. An immune-activating molecule has immunogenic activity and may evoke an immune response. In one embodiment, an immune-activating molecule relates to an antigen. In one embodiment, heat-treating of an extract reduces the presence of immune-activating molecules in an extract and/or reduces their immunogenic potential.

The term "complement system", as used herein, relates to a part of the innate immune system that enhances the ability of antibodies and phagocytic cells to clear damaged cells from an organism. More than thirty proteins are involved in the complement system. The complement system is capable of triggering different immune functions, such as phagocytosis by opsonizing antigens, inflammation by attracting macrophages and neutrophils, and membrane attack. In one embodiment, an extract for use of the present invention is advantageous in that complement has been inactivated by heat-treatment. In one embodiment, a heat-treated extract of the present invention, such as a heat-treated allogeneic extract, evokes a smaller immunogenic and/or anti-inflammatory response in a patient than an extract that is not heat-treated. In one embodiment, an extract of the present invention effectively promotes wound healing in a patient. In one embodiment, the heat-treatment of an extract of the present invention reduces its immunogenicity and therefore allogeneic extracts, such as pooled allogeneic extracts, are effectively used to enhance wound healing. In one embodiment, when referring to "complement", proteins of the complement system are meant.

The term "tissue inflammation" or "inflammation", as used herein, relates to a biological response of body tissues to harmful stimuli, such as pathogens, damaged cells, or irritants. Inflammation can be classified as either acute or chronic. The five cardinal signs of inflammation are heat, pain, redness, swelling, and loss of function. Acute inflammation is the initial response of the body to harmful stimuli and is achieved by the increased movement of plasma and leukocytes from the blood into the injured tissues. Chronic inflammation leads to a progressive shift in the type of cells present at the site of inflammation, such as mononuclear cells, and is characterized by simultaneous destruction and healing of the tissue from the inflammatory process.

The term "tissue lesion", as used herein, relates to a damage or an abnormal change in the tissue of an organism, usually caused by disease or trauma. A tissue lesion may refer to, for example, a soft-tissue lesion, a bone lesion, a brain lesion, a skin lesion, a gastrointestinal lesion, or an endodermal lesion. For example, a tissue lesion may refer to a wound, bruising, or a malignant or benign cancer site.

The term "tissue injury", as used herein, relates to a damage of a tissue, such as a damage of muscles, ligaments, bones, or tendons. Common soft tissue injuries typically occur from a sprain, strain, or external impact resulting in a contusion or overuse of a particular part of the body. Tissue injuries can result in pain, swelling, bruising and loss of function.

The term "musculoskeletal tissue", as used herein, relates to an organ system that provides form, support, stability, and movement to the body. It is made up of the bones of the skeleton, muscles, cartilage, tendons, ligaments, joints, and other connective tissue that supports and connects tissues and organs. In one embodiment, an extract of the present invention promotes wound healing in a musculoskeletal tissue and/or skin.

The term "blood", as used herein, relates to a body fluid that delivers necessary substances such as nutrients and oxygen to the cells. In vertebrates, blood is composed of blood cells suspended in blood plasma. Plasma contains mostly water, i.e. 92% by volume, and further contains proteins such as albumin, glucose, mineral ions, hormones, carbon dioxide, and blood cells. The blood cells are mainly red blood cells (RBCs), white blood cells (WBCs) and platelets. In one embodiment, blood refers to autologous and/or allogeneic blood.

The term "platelet" or "thrombocyte", as used herein, refers to a component of blood which reacts to bleeding from blood vessel injury by clumping, thereby initiating a blood clot. Structurally the platelet can be divided into four zones. The peripheral zone is rich in glycoproteins required for platelet adhesion, activation, and aggregation, such as GPIb/IX/X; GPVI; GPIIb/IIIa. The sol-gel zone is rich in microtubules and microfilaments, allowing the platelets to maintain their discoid shape. The organelle zone is rich in platelet granules. The membranous zone contains membranes derived from megakaryocytic smooth endoplasmic reticulum organized into a dense tubular system which is responsible for thromboxane A2 synthesis. This dense tubular system is connected to the surface platelet membrane to aid thromboxane A2 release.

Platelets contain dense granules, lambda granules and alpha granules. Activated platelets secrete the contents of these granules through their canalicular systems to the exterior. Alpha granules contain components such as P-selectin, platelet factor 4, transforming growth factor-β1, platelet-derived growth factor, fibronectin, B-thromboglobulin, vWF, fibrinogen, and coagulation factors V and XIII. Delta or dense granules contain components such as ADP or ATP, calcium, and serotonin. Gamma granules contain components such as several hydrolytic enzymes. Lambda granules contain components such as components involved in resorption during later stages of vessel repair. In one embodiment, alpha granules in blood platelets contain growth factors PDGF, IGF-1, EGF, and TGF-β which begin healing of wounds by attracting and activating macrophages, fibroblasts, and endothelial cells.

Platelets release various stimulating factors such as platelet-derived growth factor (PDGF), a potent chemotactic agent, and TGF beta, which stimulates the deposition of extracellular matrix, fibroblast growth factor, insulin-like growth factor 1, platelet-derived epidermal growth factor, and vascular endothelial growth factor. In one embodiment, an platelet-derived extract of the present invention is enriched in growth factors and/or cytokines released by platelets, compared to growth factor and/or cytokine levels in whole blood.

The term "administering", as used herein, relates to the application of a composition such as a platelet-drived growth factor extract or a pharmaceutical preparation comprising an extract to a patient in need thereof. In one embodiment, said extract or pharmaceutical preparation is administered intratendinously, intramuscularly, intraligamentarily, intraosseously, intragastrointestinally, intraperitoneally, orally, nasally, mucosally, intrabronchially, intrapulmonarily, intradermally, intracoronarily, subcutaneously, intravenously, intravascularly, intrathecally, intraocularly, intraarticularly, or intranodally. In one embodiment, an extract or pharmaceutical preparation is co-administered with a therapeutic agent such as an anti-inflammatory drug, e.g. betamethasone or triamcinolone, or any other glucocorticoid. In one embodiment, administering refers to local, topical, and/or systemic administration. In one embodiment, a platelet-drived growth factor extract is administered into a tendon, into a joint, and/or subcutaneously. In one embodiment, an extract of the present invention is administered as a dosage in the range of 0.5 to 10 ml, preferably 1-6 ml.

An "effective amount" is an amount of the platelet-derived growth factor extract or the pharmaceutical preparation as described herein that alleviates symptoms and/or stimulates healing of the tissue defect.

The term "patient", as used herein, relates to a human or an animal, preferably a mammal such as a horse. In one embodiment, said patient is a human, a dog, a cat, a sheep, or a horse.

The term "allogeneic formulation", as used herein, relates to an extract prepared from an allogeneic blood sample. In one embodiment, an allogeneic formulation preferably relates to an allogeneic pooled heat-treated growth factor and cytokine rich concentrated plasma. In one embodiment, said formulation may further comprise any of a therapeutic agent, fibronectin, collagen, hyaluronic acid, cord blood lysate, and an excipient, or any combination thereof. In one embodiment, said fibronectin is globular and/or fibrillar fibronectin. In one embodiment, said hyaluronic acid is cross-linked or non-crosslinked.

Allogeneic preparations of PRGFC are typically superior to autologous preparations, as allogeneic preparations may be obtained from whole blood of healthy donors instead of, in the case of an autologous PRGFC, whole blood retrieved from the patients that suffer from a pathological condition such as a defect, a chronic disease, or an inflammatory condition. Furthermore, pathological conditions such as defects, chronic diseases and inflammatory conditions may affect platelet count and cytokine profile of the subject's plasma. Subjects of senior age, smokers, and subjects obtaining pharmacotherapy can also have altered plasma profiles, which may affect therapy efficiency. Many patients may not qualify for a blood donation in accordance with the WHO guidelines, thus making their blood unsuitable for the production of blood-derived therapeutics. Thus, an advantage of the present invention is that a method of preparing an allogeneic extract preparation with reduced immunogenicity is provided.

The term "growth factor", as used herein, relates to a naturally occurring substance capable of stimulating any of cellular growth, proliferation, healing, and cellular differentiation. Typically, a growth factor is a protein or a steroid hormone. In one embodiment, growth factors may comprise any of platelet-derived growth factor, transforming growth factor, platelet factor interleukin, platelet-derived angiogenesis factor, vascular endothelial growth factor, epidermal growth factor, insulin-like growth factor, and combinations of the foregoing. In one embodiment, growth factors are enriched in an extract of the present invention.

The term "therapeutic agent", as used herein, relates to a drug having a therapeutic effect. In one embodiment, a pharmaceutical preparation of the present invention comprises a therapeutic agent such as an anti-inflammatory drug, e.g. betamethasone or triamcinolone, or any other glucocorticoid.

The term "pharmaceutical preparation", as used herein, relates to a pharmaceutically acceptable form of an extract of the present invention, optionally comprising further components such as excipients, collagen, fibronectin, therapeutic agents. In one embodiment, a pharmaceutical preparation comprises a platelet-derived growth factor extract of the present invention, such as an allogeneic formulation of a platelet-derived growth factor extract. In one embodiment, a pharmaceutical preparation comprises 1:1 of hyaluronic acid (HA):extract of the present invention, such as 1 mg/ml HA in 2 ml solution combined with 2 mL extract.

The term "collagen", as used herein, relates to the main structural protein in the extracellular space in the various connective tissues in the body, and has an important role in wound healing. Collagen, such as collagen type I or collagen type II, of natural or synthetic origin may be used in a preparation of the present invention. Particularly, collagen scaffolds may be used as a support for tissue growth and cell attachment in a method of preventing or treating a tissue defect, such as in promoting wound healing. In one embodiment, collagen nanoparticles are comprised in a preparation of the present invention.

The term "cord blood lysate", as used herein, relates to a lysate of cord blood obtained from postpartum placenta after birth. The cord blood is centrifuged at minimal speed to separate plasma and red blood cells. The plasma is homogenized and subsequently centrifuged to form a pellet containing platelets. Approximately 2/3 of the excess plasma is removed and the pellet resuspended in the remaining plasma volume. The sample is then frozen at - 80 °C, thawed, and treated with ultrasound.

The term "red blood cell fraction", as used herein, relates to a fraction obtained from a blood sample which contains the red blood cells. In one embodiment, said fraction is obtained from the blood sample by centrifugation, preferably by centrifugation at from 650 g to 900 g for 1-15 min.

The term "plasma fraction", as used herein, relates to a fraction obtained from a blood sample which contains the plasma. In one embodiment, said fraction is obtained from the blood sample by centrifugation, preferably by soft spin centrifugation at from 100 g to 600 g for 1-15 min, preferably at from 100 to 500 g for about 5 min. In one embodiment, a plasma fraction can be separated into a platelet-poor plasma supernatant and a platelet-rich pellet, preferably by hard spin centrifugation at from 2900 g to 5000 g for 10 - 15 min at room temperature.

The term "providing a blood sample", as used herein, relates to obtaining a blood sample from a donor, such as an allogeneic donor, or from the patient, and providing said blood sample for preparing a platelet-derived growth factor extract. In one embodiment, a blood sample may relate to a blood sample of one individual or to a pooled blood sample from several blood donations, wherein said several blood donations may be from several donors or from one donor at several time points.

The term "homogenizing", as used herein, relates to a process whereby a biological sample is brought to a state such that all fractions of the sample are equal in composition. In one embodiment, homogenization may be performed by vortexing, pipetting, thoroughly mixing, shaking, or any other means of agitation. In one embodiment, homogenization is carried out by pooling and mixing multiple samples of PRP. In one embodiment, obtaining PRP comprises pooling of multiple samples such as multiple samples from the same donor, particularly pooling multiple extracts from the same or different donors in an allogeneic mixture after heat treatment.

The term "platelet-poor plasma supernatant", as used herein, relates to a supernatant of plasma that has been centrifuged to separate the platelets from the plasma. A platelet-poor plasma supernatant may be obtained by a PRP method, a buffy coat method, or by microfluidics. In one embodiment a PRP method comprises obtaining whole blood e.g. by venepuncture, in acid citrate dextrose (ACD) tubes, not chilling the blood before or during platelet separation, centrifuging the blood using a soft spin, transferring the supernatant plasma containing platelets into another sterile tube without anticoagulant, centrifuging the tube at a higher speed to obtain a platelet concentrate, thereby obtaining PRP, preferably the lower part of the tube, such as the lower 1/3^{rd}, and platelet-poor plasma (PPP), preferably the upper part of the tube, such as the upper 2/3^{rd}, and platelet pellets at the bottom of the tube, removing the PPP and suspending the platelet pellets in a minimum quantity of plasma such as 2-4 mL by gently shaking the tube. In one embodiment, a buffy coat method comprises obtaining and storing whole blood at 20°C to 24°C before centrifugation, centrifuging at a high speed, thereby forming a bottom layer consisting of red blood cells, a middle layer consisting of platelets and white blood cells, and a top PPP layer, removing supernatant plasma from the top, transferring the buffy coat layer to another sterile tube, centrifuging at a low speed to separate white blood cells or using leucocyte filtration filter. In one embodiment, said PRP method and/or buffy coat method comprise activating platelets with thrombin or calcium.

The term "platelet-rich pellet", as used herein, relates to a pellet which comprises the plurality of platelets of a blood sample. A platelet-rich pellet is preferably obtained by centrifugation of PRP or platelet concentration at from 100 g to 10000 g, preferably 2800 g to 6000 g, for 1 min to 60 min, preferably for 15 min to 30 min. In one embodiment, after centrifugation for obtaining a platelet-rich pellet, the excess plasma is removed and the pellet is resuspended prior to freeze-thawing.

The term "releasing cytokines and growth factors", as used herein, relates to a process in which cytokines and growth factors are released from platelets. In one embodiment, platelets are activated and/or are lysed during a cycle of freezing and thawing a sample comprising platelets, which results in the release of cytokines and growth factors from said platelets. In one embodiment, cytokines and growth factors may be released from platelets by activation and/or lysis through multiple freeze-thaw-cycles, electroporation, ultrasound treatment, chemical lysis, treatment with CaCl₂, activation with calcium and/or thrombin,

The term "deactivating immune-activating molecules", as used herein, relates to reducing the immunogenic potential of an extract. In one embodiment, the immunogenic potential of an extract of the present invention is reduced by heat-treating said extract. In one embodiment, said heat-treatment reduced the amount of immune-activating molecules and/or reduces their immunogenic potential. In one embodiment, said heat-treatment inactivates components of the complement system. In one embodiment, a platelet lysate is heat-treated, so that proteins of the complement system and other undesired proteins coagulate, and the coagulates may be removed using filtration, after the sample has been cooled down to about 4°C.

The term "obtaining heat-treated platelet-derived growth factor extract", as used herein, relates to obtaining a heat-treated extract of blood such as in form of a solution, dispersion, suspension, or lyophilized powder. The extract is for use in a method of treating a tissue defect such as a tissue inflammation or a tissue lesion, said lesion for example deriving from a tissue injury and/or from a surgical procedure such as an organ transplantation. An extract can be formulated in to various pharmaceutically acceptable formulations to provide a pharmaceutical preparation comprising said extract. In one embodiment, an extract is preferably an allogeneic growth factor and cytokine concentrated plasma extract. In one embodiment, a liquid extract is obtained and provided to a patient in need thereof, optionally in combination with HA, such as pre-mixed HA. In one embodiment, obtained extract is frozen and/or lyophilized for storage and later use. In one embodiment, frozen extracts were stored at -20 °C for up to one month. In one embodiment, frozen extracts were stored at about -80 °C for more than one month.

The term "sterile filtration", as used herein, relates to sterilization of an extract of the present invention by microfiltration using a membrane filter. A microfilter with pore size 0.2 µm will typically effectively remove microorganisms. Nanofilters with a smaller pore size (20-50 nm) are typically used to remove viruses.

The term "pooled blood sample", as used herein, relates to a blood sample which comprises blood from several donors and/or from one donor obtained at several time points and/or body sites. A pooled blood sample is a mixture of different blood samples. Obtaining extracts from pooled blood samples instead of from individual blood samples allows for higher reproducibility and standardization of the obtained platelet-derived growth factor extracts, as well as allows for obtaining greater amounts of extract. Alternatively, heat-treated extracts of different blood samples may first be prepared individually, and then these heat-treated extracts are pooled.

The term "shock-freezing", as used herein, relates to quickly freezing a sample. Typically, the samples are subjected to temperatures well below the freezing point of water, such as with liquid nitrogen, dry ice, or freezer having a temperature of about - 80°C.

The term "ultrasound treatment", as used herein, relates to applying ultrasound to a sample, typically very high frequency sound waves between 800,000 Hz and 2,000,000 Hz. In one embodiment, ultrasound is applied to a priorly frozen sample in an ultrasound water bath at about 37°C until ice and clothes are dissolved.

The term "grade", as used herein, relates to a staging of lameness. A lameness grading system has been developed by the American Association of Equine Practitioners (AAEP) to aid both record-keeping of horse lameness which has been defined as any alteration of the horse's gait. The scale ranges from zero to five, with zero being no perceptible lameness, and five being most extreme. The AAEP guidelines explain the grading system this way:
0: Lameness not perceptible under any circumstances.
1: Lameness is difficult to observe and is not consistently apparent, regardless of circumstances (e.g. under saddle, circling, inclines, hard surface, etc.).
2: Lameness is difficult to observe at a walk or when trotting in a straight line but consistently apparent under certain circumstances (e.g. weight-carrying, circling, inclines, hard surface, etc.).
3: Lameness is consistently observable at a trot under all circumstances.
4: Lameness is obvious at a walk.
5: Lameness produces minimal weight bearing in motion and/or at rest or a complete inability to move.

### BRIEF DESCRIPTION OF THE FIGURES

The present invention is now further described by reference to the following figures.

All methods mentioned in the figure descriptions below were carried out as described in detail in the examples.

In the following, reference is made to the examples, which are given to illustrate, not to limit the present invention.

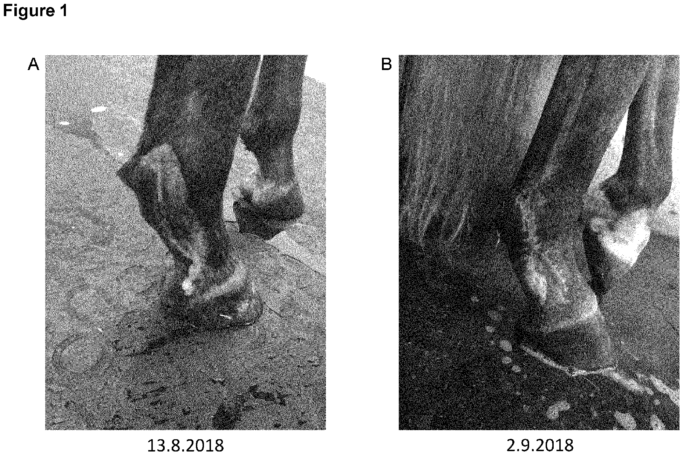

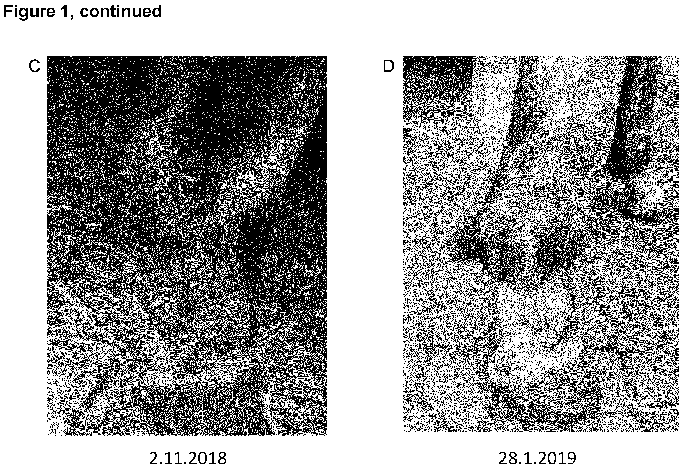

Figure 1 shows case report no. 1.
   Subject: Prika, 6 years old mare
   Diagnosis: Laceration cutis, subcutis et musculi in regio MT3-ph.1,2 lat. posttraumatica (according to the standard latin anatomical classification of equine tarsal joint regions; MT 3 - third metatarsal bone, Ph 1,2 - first and second phalangeal bone), arthritis septica MT3-ph.1.
   A) On 13.8.2018, injury on right hind leg occurred with a wound being 37 cm long and 15 cm wide. The horse was accepted into veterinary care. Revision was performed under general anaesthesia. Arthroscopy and joint lavage of the septic joint and debridement of damaged tissue was performed with lavage of the wound area. Next, muscles and subcutis were sutured and skin was clamped. Before clamping, subcutaneous tissue at the wound edges was infiltrated with 2 ml APGFCCP on every 2 cm of wound edge. The wound was dry bandaged daily. Parenteral therapy: gentamycin 6,6 mg/kg 24h i.v., Ceftiofur - Na 2,2 mg/kg 12 h i.v., Flunixin 1mL/45kg 24h i.v.; 10 days.
   B) On 2.9.2018, the clamps were removed. The upper half of the wound showed closing of the wound. The lower half of the wound was covered with fibrous tissue and would healing per secundam. The wound was bandaged on daily basis and an ointment with Derma Gel® was applied for wound surface care. The fibrous tissue in the open part of the wound was removed every 6 weeks in the course of the healing process in order to enable normal skin overgrowth.
   C) On 2.11.2018, the wound was almost closed completely, with only a minor area of fibrous tissue. The wound was dry and the upper 2/3 of the wound were fully closed. No further cutting was performed, since healing was successful and the skin overgrew the defect.
   D) On 28.1.2019 photo of completely healed leg was taken, no cutting was preformed post third control; only minimal scarring remained.

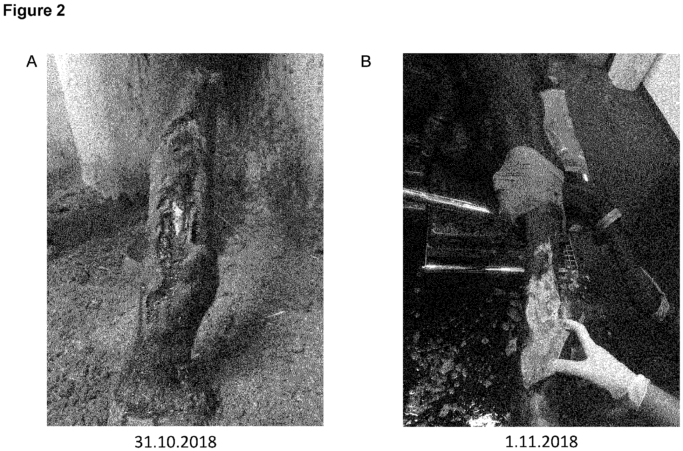

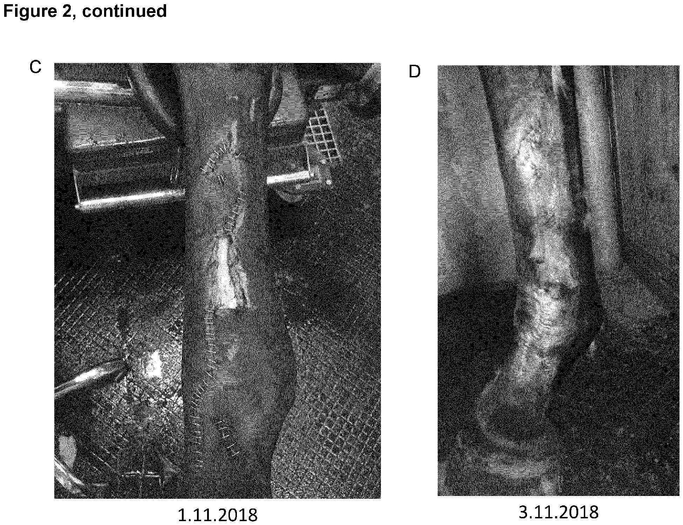

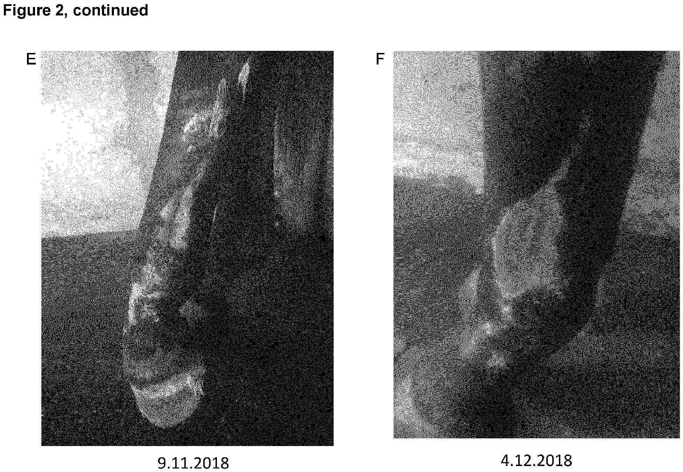

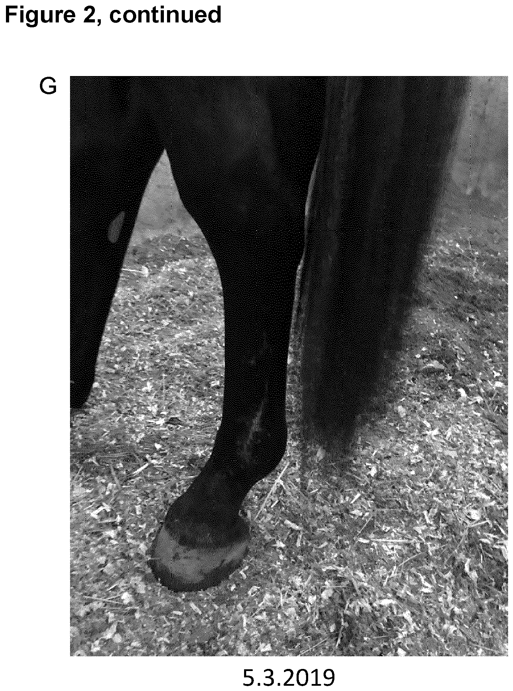

Figure 2 shows case report no. 2.
   Subject: Diamant D'amur, 12 months old, male
   Diagnosis: Laceratio cutis, subcutis et musculi in regio MT3-ph.1 lat.
   A) On 31.10.2018, an injury on the left hind leg occurred with the wound being 35 cm long and 12 cm wide, and with hindered flexion of the hoof resulting from a muscle damage. The horse was treated the next day. A wound revision was performed under general anaesthesia.
   B) On 1.11.2018, arthroscopy and joint lavage of the septic joint were performed, as well as debridement of damaged tissue with lavage of wound area. Next, the muscles and the subcutis were sutured and the skin was clamped. Before clamping, subcutaneous tissue at the wound edges was infiltrated with 2 ml APGFCCP on every 2 cm of wound edge. Wound was dry bandaged on a daily basis.
      Parenteral therapy: gentamycin 6,6 mg/kg 24h i.v., Ceftiofur - Na 2,2 mg/kg 12h i.v., Flunixin 1mL/45kg 24h i.v.; 10 days.
   C) On 1.11.2018, in the centre of the wound, a full-thickness tissue defect measuring 10 cm in length and 2,5 cm in width remained open with bone left exposed. The aim of the APGFCCP administration was to accelerate healing of proximal soft tissue to cover the bone in short as possible time period. Exposure of bone tissue to the environment is a high-risk factor for infection and consequential osteomyelitis, which would leave no other option other than euthanasia of the animal.
   D) The wound was bandaged on a daily basis and soft tissue started covering the bone.
   E) The wound was bandaged on a daily basis. Granulation tissue was filling the defect above the bone, said bone being fully covered 14 days after the injury occurred. The fibrous tissue was removed every 6 weeks to enable full-thickness healing and skin overgrowth.
   F) Last check-up with cutting. The wound was almost fully filled with tissue, enabling skin overgrowth and closure of the wound. Afterwards, the skin overgrew the remaining area of the defect.
   G) Photography from 5.3.2019 shows completely healed leg, with minimal scar. No cutting was preformed post third control.

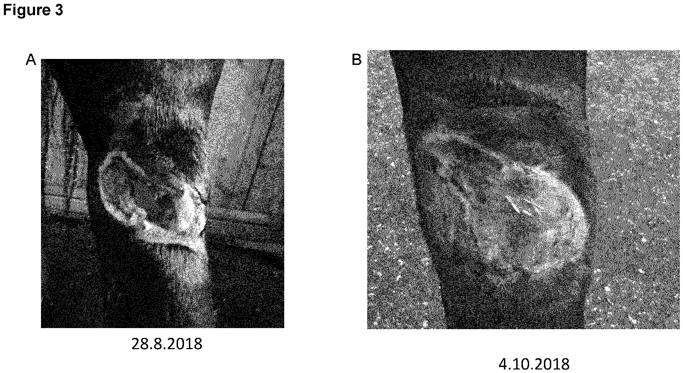

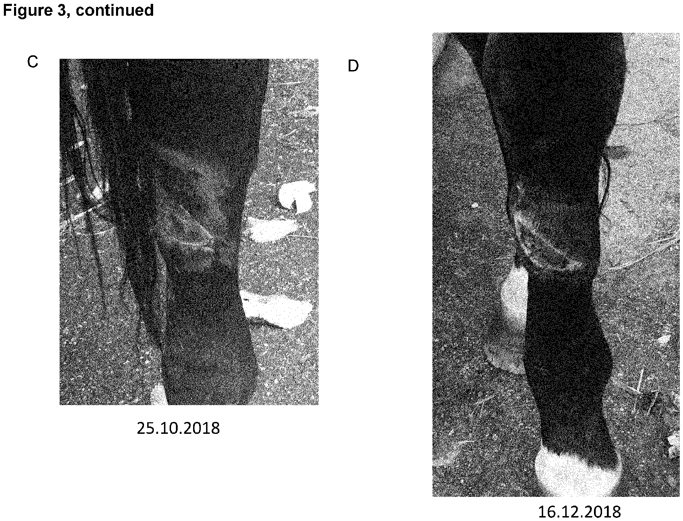

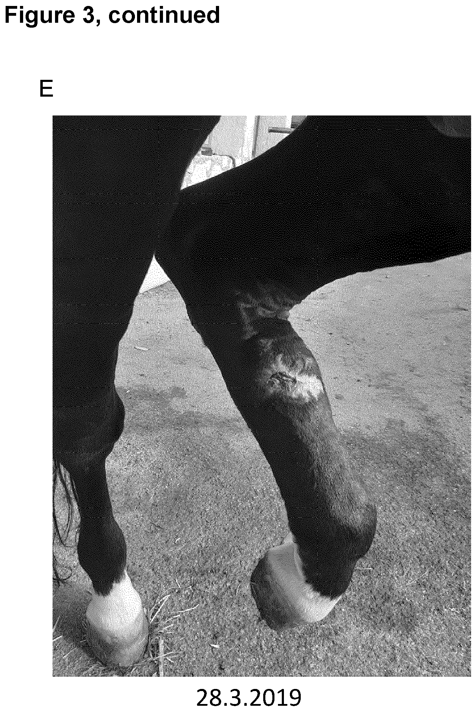

Figure 3 shows case report no. 3.
   Subject: Vegas, 2 years old stallion
   Diagnosis: Laceration cutis, subcutis et musculi in regio MT3 dorsomedialis posttraumatica.
   A) On 28.8.2018, an injury on the left hind leg occurred, the wound being 20 cm long and 10 cm wide. The horse was accepted into veterinary care. Revision was performed under general anaesthesia. Debridement of damaged tissue was performed with lavage of wound area. Subcutaneous tissue at the wound edges was infiltrated with 2 ml APGFCCP on every 2 cm of wound edge. The injury left no tissue to close the full-thickness defect, leaving the bone exposed. The wound was dry bandaged on a daily basis.
      Parenteral therapy: gentamycin 6,6 mg/kg 24h i.v., Ceftiofur - Na 2,2 mg/kg 12h i.v., Flunixin 1mL/45kg 24h i.v.; 10 days.
   B) Tissue proliferation closed the defect in full-thickness within 3 weeks after injury. Daily lavage of the wound with saline solution was performed with bandaging. Fibrosis was removed every 6 weeks, twice during healing process to enable normal skin overgrowth.
   C) Dermal tissue started to overgrow the site of the defect with edges closing together (25.10.2018). Daily lavage of the wounded area with saline solution was performed with bandaging.
   D) Last check-up with cutting. The wound was dry. After this, skin started to cover the remaining defect until fully covered.
   E) On 28.3.2019, wound was completely healed, with portion of scar tissue remaning; no cutting was preformed post third control.

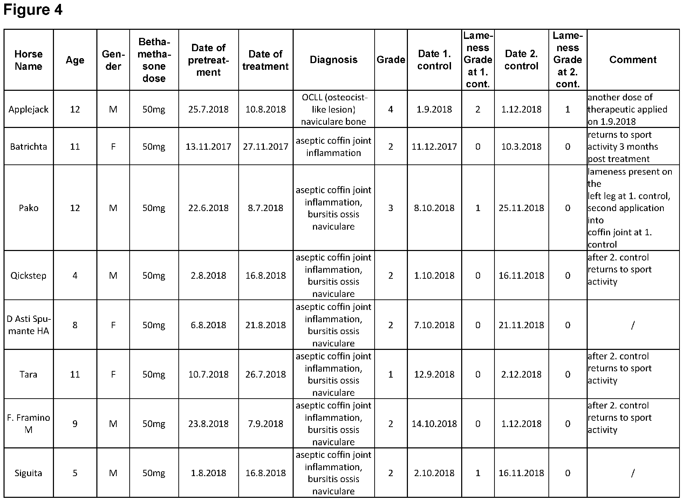

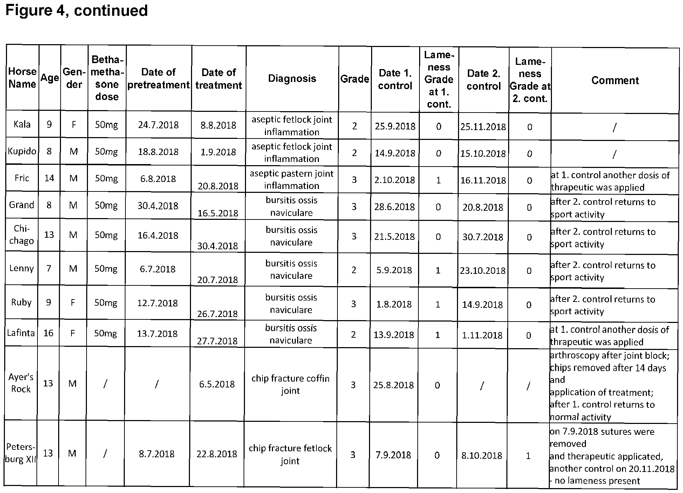

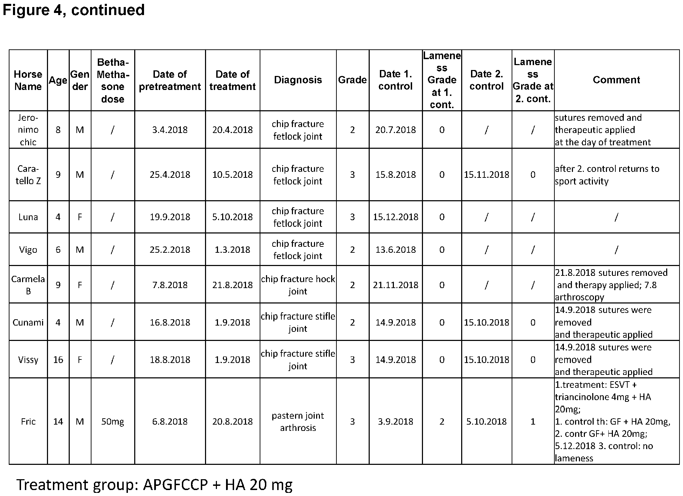

Figure 4 shows the data obtained with 26 horses having a musculoskeletal defect which were treated with the platelet-derived growth factor extract for use of the present invention in addition to hyaluronic acid. The treated defects showed surprisingly good healing behavior. At the time point of the first control, each horse showed a significant improvement compared to the initial condition. Each horse showed an improvement of the health condition of at least one grade in accordance with the parameters of the American Association of Equine Practitioners (AAEP) Lameness Scale (grading from 0 to 5).
   At the time point of the second control, almost all horses showed grade 0 at the defect site and only three horses exhibited a defect grade >0. These three horses each had a defect grade 1 at the time point of the second control which represented an amelioration of the defect condition of at least 2 grade levels. "Ctrl"/"Cont"/"Con" represent "control".

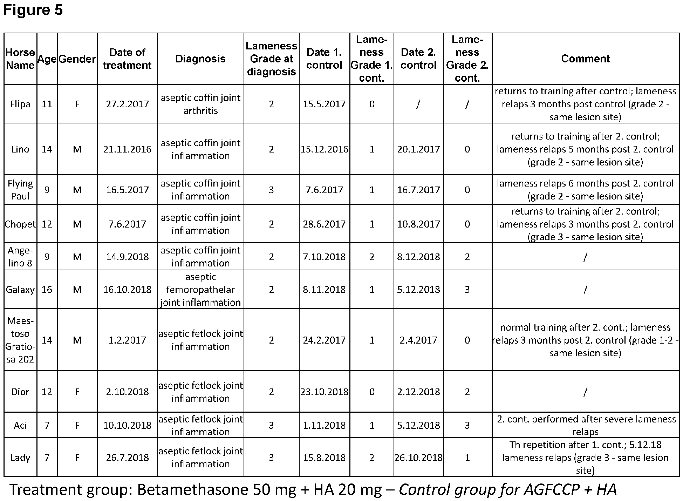

Figure 5 shows the data obtained with 10 horses having a musculoskeletal defect which were treated with betamethasone (flosteron) and hyaluronic acid (HA). The control group shown in figure 5 comprises horses treated with flosteron and HA only. In those control cases, recovery was at most temporarily and insufficient, and lameness relapsed after a certain period of time, typically 3-6 months after maximum recovery. "Sc" represents "subcutaneously", "Th" represents "therapy". "Ha 20 mg cont." represents hyaluronic acid 1% in 20 mg solution.

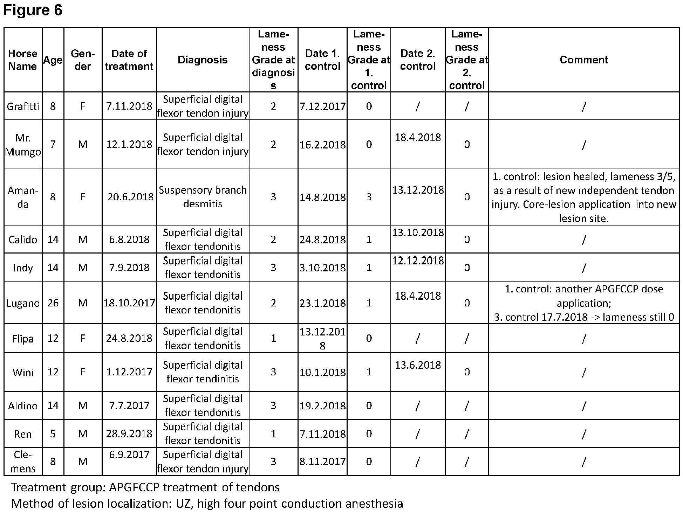

Figure 6 shows the data obtained with 11 horses having a musculoskeletal defect which were treated with the platelet-derived growth factor extract for use of the present invention. The treated defects showed surprisingly good healing behavior.
   At the time point of the first control, more than 50 % of the horses (6 of 11) showed complete recovery indicated by grade level o. At the time point of the second, the remaining 5 horses also showed complete recovery indicated by grade level o. All horses that reached 0 grade are deemed cured with complete recovery. Almost all subjects treated achieved full recovery after a certain period of time, typically 1 to 6 months after treatment with regard to tendonitis, and 2 weeks to 6 months after treatment with regard to degenerative arthritis. "UZ" represents "ultrasound". Even cases of arthrosis were successfully treated at a late stage of the condition.

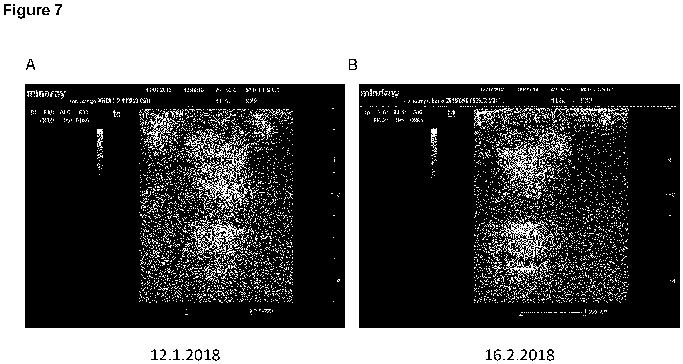

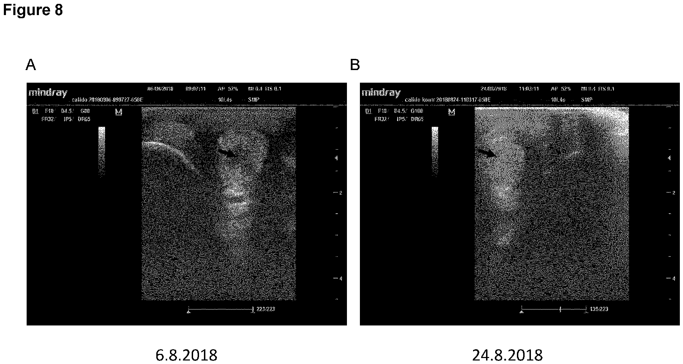

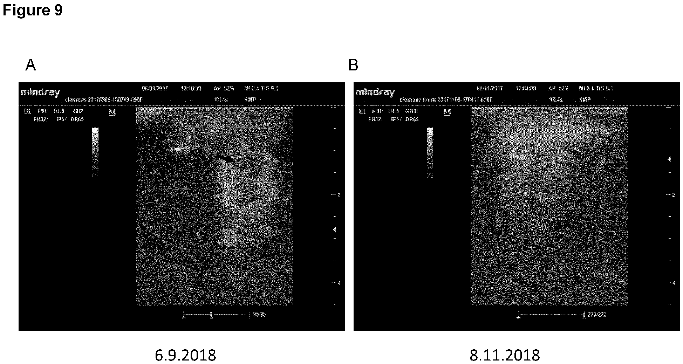

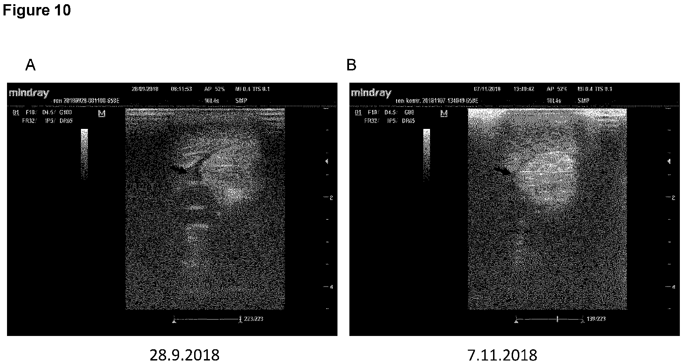

Figures 7 - 10 represent ultrasound pictures, with shown hypoechogenic lesions at the diagnosis (A) and completely healed defects at the first control (B) post platelet-derived extract application. Following cases are represented: Figure 7 - Mr. Mungo, Figure 8 - Calido, Figure 9 - Clemens and Figure 10 - Ren.
   All cases are described in Figure 6 (APGFCCP treatment of tendons). Lesion sites are marked with a marker.

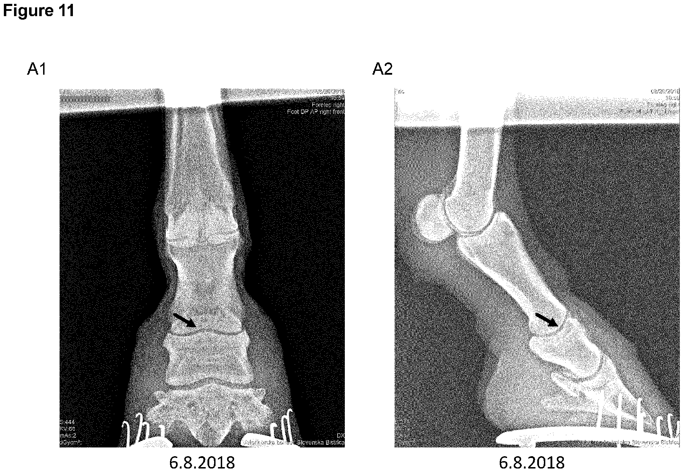

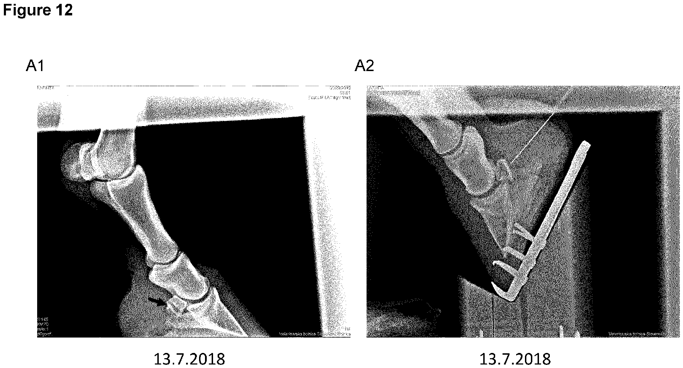

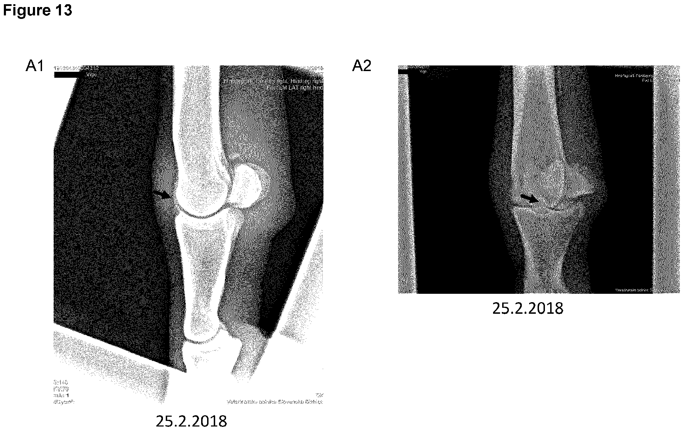

Figures 11 - 13 represent X-ray pictures of cases of treatment of joints, using APGFCCP with 20 mg hyaluronic acid (1%) (APGFCCP + HA). Cases shown are following: Figure 11 - Fric, Figure 12 - Lafinta, Figure 13 - Vigo.
Figure 11 represents case of pastern joint arthrosis, a final stage of degenerative arthritis, with vast degenerative changes of carthilage. After three applications of APGFCCP with HA, the patient exhibited no lameness after third control on 5.12.2018.
Figure 12 represents persistent bursitis of navicular bone, which was fully treated after seond therapeutic application at first control post initial APGFCCP + HA treatment.
Figure 13 represents case of treatment of fetlock joint inflammation, resulting from chip fracture. The pain was absent at first control post treatment.
Figure 14 shows a schematic of a preparation protocol of APGFCCP, with a description of all included steps in the process.

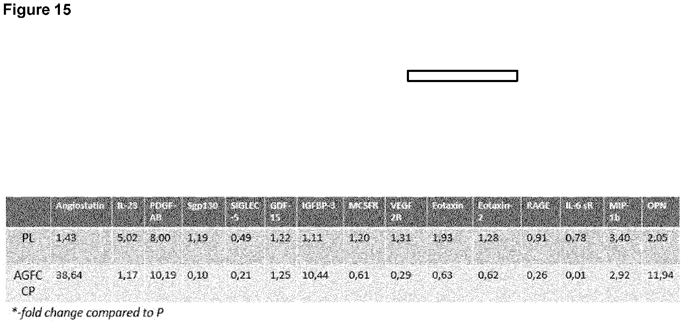

Figure 15 shows a growth factor and cytokine content comparison between allogeneic plasma (P), allogeneic platelet lysate (PL) and AGFCCP. Depicted are fold-changes in the cytokine protein levels of PL and AGFCCP compared to P. 13 significant biomarker changes were observed in AGFCCP compared to control (allogeneic human plasma), namely changes inOPN, angiostatin, IL-23, PDGF-AB, sgp130, Siglec-5, GDF-15, IGFBP-3, MCSFR, VEGF R2, eotaxin, eotaxin-2, RAGE, IL-6 sR, IL-16, MIP-ib. Furthermore, notable biomarker differences between PL and AGFCCP were observed with regard to the markers mentioned above, and additionally with regard to EG-VEGF, FGF-7, SCF R, IL-6, and VEGF-C. The extract of the present invention, AGFCCP, showed a shift in the levels of proteins relevant for wound healing towards a wound healing- stimulating environment compared to plasma.

### EXAMPLES

### Example 1:

### Blood donation

Human blood donors were selected based on World Health Organization's (WHO) Guidelines on Assessing Donor Suitability for Blood Donation, with additional requirements that donors must not be smokers and must be younger than 27 years, preferably female. As evidence suggests, platelet counts decrease with age [7]. Consequentially, younger donor's platelet-rich plasma results in higher growth factor concentrations after lysis of platelets. Whole blood was collected from 8 human donors, aged from 18 to 25 years.

Cord blood was obtained from postpartum placenta immediately after birth. All donors donated three 6 mL Vacuette™ ACD-iA containing eprouvettes of whole blood.

Horse blood from healthy horse donors was collected in 600 mL blood bags containing 40 mL ACD-iA. Whole blood was collected from multiple healthy donors at regular blood analysis, aged from 5 months to 6 years of both genders. Owner consent was obtained prior blood collection.

### Preparation of Allogeneic Growth Factor and Cytokine Concentrated Plasma

Donated blood was transferred to 50mL falcon tubes and centrifuged at 710 x g for 3 min RT with minimal speed for separation of plasma and red blood cells. Plasma was then homogenized and transferred to new falcon tubes, and centrifuged again at 4500 rpm for 10 min at RT to form a pellet containing platelets. Platelets were then concentrated by removing at least two-thirds of the entire plasma volume. Excess plasma was transferred to new falcon tubes for isolation of fibronectin, or discarded. The pellet was resuspended in the remaining plasma volume using a vortexer. For platelet concentrated plasma (PCP), samples were transferred to smaller 2 mL tubes and centrifuged.

PCP was then frozen at -80°C for at least 5 hours. PCP samples were thawed and treated with ultrasound in an ultrasound water bath at 37°C until ice and clots were dissolved. Obtained platelet lysate was then heated for 30 min at 56°C. Post heating, samples were put in the cold room at 4 °C overnight to settle, and then centrifuged at 4500 rpm for 10 min at RT in order to remove debris. Samples from different donors were pooled and homogenized. Sterile filtration was performed to ensure removal of any impurities left. Obtained APGFCCP was transferred to a syringe for therapeutic application and stored at -20°C until use.

Cord blood platelet lysate was prepared by the same previously described protocol, but was not heat-treated due to the lack of immunogenic properties.

The following therapeutic preparations were prepared:
- APGFCCP
- APGFCCP + cord blood platelet lysate
- APGFCCP + hyaluronic acid (hyaluronan)
- APGFCCP + fibronectin nanofibers
- APGFCCP + fibronectin nanofibers + hyaluronic acid (hyaluronan)
- APGFCCP + fibronectin gel + fibronectin nanofibers
- APGFCCP + fibronectin gel + hyaluronic acid (hyaluronan)
- APGFCCP + fibronectin gel + hyaluronic acid (hyaluronan) + fibronectin nanofibers

Umbilical cord platelet lysate was shown to contain higher contents of proteins and growth factors than peripheral blood platelet lysate and expressed ability to enhance proliferation of human mesenchymal stem cells (8). 1% of cord blood-derived platelet lysate was added to the APGFCCP to further facilitate healing abilities of the invention.

### Preparation of formulation with hyaluronic acid

In therapeutic applications for joint conditions, APGFCCP was mixed with a viscoelastic solution of 10 mg 1% sodium hyaluronate (HA) (Synocrom®, Croma-Pharma) in a ratio of 1:1. Affected joints of patients with OA have dramatically decreased viscoelasticity of synovial fluid. Consequentially, mechanical stress on joints and affected joint surfaces is increased, causing pain, stiffness and inflammation while moving. A viscoelastic hyaluronic acid solution lubricates joint surfaces, decreases friction, reduces inflammation and enhances healing effect of APGFCCP in intraarticular injection.

### Immunological analysis

IgG, IgM, IgE and all three paths of complement activation (classical, alternative and lectine) were measured to evaluate immunological parameters. Multiple substances and formulations were tested in order to clearly distinguish between immunogenic potential of them. The substances tested and compared were:
- control (saline)
- allogeneic pooled plasma
- allogeneic pooled platelet lysate
- APGFCCP.

### Example 2:

### Treatment

Horses suffering from clinical lameness of mild to severe form in any joint were included in to the study. A broader spectrum of equine patients was included in order to evaluate the therapeutic effect of a platelet-derived growth factor extract of the invention. The source of the lameness of each equine patient was determined and confirmed by both local analgesia and a positive flexion test. The lameness was evaluated in linear and circular motion on hard and soft ground. Lameness was graded from 0 (no lameness) to 5 (minimal weight bearing lameness) according to American Association of Equine Practitioners (AAEP). Furthermore, radiographic (X-ray), joint effusion, and ultrasound (UZ) imaging was performed to clarify the clinical condition. All diagnostic and control procedures were performed by the same veterinarian.

The following treatments were performed:
a) 11 horses diagnosed with tendon injury were treated with 2 mL APGFCCP core lesion application right after diagnosis confirmation. Some cases were treated for multiple lesions on different anatomical locations (cf. Figure 6).
b) 10 horses diagnosed with aseptic arthritis were treated by injection with 50 mg betamethasone (Flosteron, Krka) intraarticularly, and subsequently, after approximately 14 days, with 4 mL of APGFCCP/HA (1:1) intraarticularly (cf. Figure 5).
c) 10 horses diagnosed with a similar aseptic arthritis were treated with 50mg betamethasone + 2 mL HA intraarticularly alone (group used as control).
d) 11 horses were diagnosed with bursitis and treated with 4 mL of APGFCCP/HA (1:1) injected intrabursally (cf. Figure 4).
e) 2 chip fractures in joints were diagnosed and treated with 4 mL of APGFCCP/HA (1:1) injected intraarticularly (cf. Figure 4).
f) 1 osteocyst-like lesion (OCLL) was treated with 4 mL of APGFCCP/HA (1:1) injected intrabursally (cf. Figure 4).
g) 1 case of pastern joint arthrosis was treated using 4 mL of APGFCCP/HA (1:1), injected intraarticularly (cf. Figure 4).

In some cases, horses were diagnosed with multiple orthopedic conditions (cf. Figure 4). Additionally, 3 cases of severe wounds, treated with APGFCCP injections were studied.

First control was preformed within 14 days to 6 months post treatment and second control 2 months to 6 months post treatment respectively after treatment with APGFCCP.

### Results

Treated horses were typically first evaluated 14 days to 6 months post therapeutic application of APGFCCP, or its formulations, and 3 months prior return to regular activity, deviations in evaluation times occurred because all horses were owner horses kept in private facilities.

Subjects in control group were evaluated 3 weeks post application of 50 mg betamethasone, optionally treated with hyaluronic acid (1mg/mL) in a formulation with 50 mg betamethasone, and 3 months before return to regular activity. Control examinations were performed earlier in cases of worsening of symptoms. The treatment group was compared with control group of 10 horses, affected with similar aseptic arthritic conditions treated with betamethasone only (Fig 5). Betamethasone only treatment initially showed improvement of overall condition, but with limited duration of the effect. Subjects in the control group had to cease regular training, typically 3-6 months post treatment, as a result of lameness and pain relapse, and/or worsening of symptoms.

Horses with full-thickness traumatic wounds treated using APGFCCP infiltration showed faster proliferation of functional tissue than horses without APGFCCP infiltration, which resulted in decreased need for fibrous tissue cutting. Typically, fibrous tissue is removed to enable normal healing and formation of functional tissue in full thickness with regard to defect and skin overgrowth. In usual cases fibrous tissue removal is performed twice per month, compared to once per 6 weeks in treatment cases. The present inventors show that even severe wounds can be successfully treated using an extract, preferably an allogeneic heat-treated extract, of the present invention.

APGFCCP/HA treated subjects showed permanent improvement of the overall condition, with no recurrence or worsening of symptoms of lameness or pain in any of the treated horses. Treated cases demonstrated effectiveness of APGFCCP treatment and overall long-term improvement of conditions outcomes in general and compared to relevant controls.

The platelet-derived growth factor extract of the present invention was also shown to be especially effective in treatment of tendon injury; lesions completely healed in all cases of the treatment group with horses being able to return to regular activities.

Furthermore, the effectiveness of the platelet-derived growth factor extract of the present invention in the treatment of deep wounds was proven, as indicated by lower proliferation of fibrous tissue and faster growth of skin in treated cases resulting in reduced need for scar tissue cutting.

The effect of APGFCCP on acceleration of tissue proliferation is especially useful for wounds, in which healing per primam is not possible as a result of the tissue defect. Moreover, acceleration of tissue proliferation proximal to exposed bone reduced the exposure time of bone and other tissue, and reduced the potential need for prolonged antimicrobial therapy which might result in microbial resistance to the applied antibiotics.

### Example 3:

A quantitative analysis of the cytokine contents of allogeneic plasma (P), platelet lysate (PL) and AGFCCP samples was performed using a human cytokine array, particularly Human Cytokine Array GSH-CAA-4000-1 (Bio-Rad). Allogeneic human plasma was used as control. The tested samples were prepared from whole blood by PRP and/or buffy coat (BC) method. A relative comparison between allogeneic plasma (P), allogeneic platelet lysate (PL) and AGFCCP was performed, namely the signals obtained using PL and AGFCCP were compared against the signals obtained using plasma. Two sets of samples were prepared, one set based on a PRP method, and the other set based on a buffy coat method. Only those cytokine level patterns were taken into account which were observed in the samples of both sets. Blood from the same donors was used in each category. The data was normalized and analyzed taking into account a limit of detection (LoD) and the signal strength. "Significance" was defined as above 2 for overdetected proteins and under 0,5 for underdetected proteins. 13 significant biomarker changes were observed in AGFCCP, namely changes in OPN, angiostatin, IL-23, PDGF-AB, sgp130, Siglec-5, GDF-15, IGFBP-3, MCSFR, VEGF R2, eotaxin, eotaxin-2, RAGE, IL-6 sR, IL-16, MIP-1b (Figure 15). Furthermore, there were notable biomarker differences between PL and AGFCCP in OPN, angiostatin, IL-23, PDGF-AB, sgp130, Siglec-5, GDF-15, IGFBP-3, MCSFR, VEGF R2, eotaxin, eotaxin-2, RAGE, IL-6 sR, IL-16, MIP-ib, EG-VEGF, FGF-7, SCF R, IL-6, and VEGF-C (Figure 15). The extract of the present invention showed a shift in the levels of proteins relevant for wound healing towards a wound healing- stimulating environment compared to plasma.

### REFERENCES

[1] Pincus T, Kent P, Bronfort G, et al. Twenty-five years with the biopsychosocial model of low back pain-is it time to celebrate? A report from the twelfth international forum for primary care research on low back pain. Spine (Phila Pa 1976) 2013;38: 2118-23.
[2] Svege I, Nordsletten L, Fernandes L, et al. Exercise therapy may postpone total hip replacement surgery in patients with hip osteoarthritis: a long-term follow-up of a randomised trial. Ann Rheum Dis 2013. Published Online First 20 Nov 2013.
[3] Hudgens JL, Sugg KB, Grekin JA, Gumucio JP, Bedi A, Mendias CL. Platelet-Rich Plasma Activates Proinflammatory Signaling Pathways and Induces Oxidative Stress in Tendon Fibroblasts. Am J Sports Med. 2016;44(8):1931-40.
[4] Kia C, Baldino J, Bell R, Ramji A, Uyeki C, Mazzocca A. Platelet-Rich Plasma: Review of Current Literature on its Use for Tendon and Ligament Pathology. Curr Rev Musculoskelet Med. 2018;11(4):566-572.
[5] Hudgens JL, Sugg KB, Grekin JA, Gumucio JP, Bedi A & Mendias CL. Platelet-Rich Plasma Activates Proinflammatory Signaling Pathways and Induces Oxidative Stress in Tendon Fibroblasts. The American Journal of Sports Medicine, 2016; 44(8), 1931-1940.
[6] Anitua E, Muruzabal F, De la Fuente M, Merayo-Lloves J, Orive G. Effects of heat-treatment on plasma rich in growth factors-derived autologous eye drop. Experimental Eye Research, Volume 119. 2014; Pages 27-34.
[7] Klatte-Schulz, F.; Schmidt, T.; Uckert, M.; Scheffler, S.; Kalus, U.; Rojewski, M.; Schrezenmeier, H.; Pruss, A.; Wildemann, B. Comparative Analysis of Different Platelet Lysates and Platelet Rich Preparations to Stimulate Tendon Cell Biology: An In Vitro Study. Int. J. Mol. Sci. 2018, 19, 212.
[8] Wang, Qian, et al. "Identification of a Central Role for Complement in Osteoarthritis." Nature Medicine, U.S. National Library of Medicine, 6 Nov. 2011, www.ncbi.nlm.nih.gov/pmc/articles/PMC3257059/.
[9] Chantre C., Campbell P., Golecki H., Buganza Tepole A., et al. (2018). Production-scale fibronectin nanofibers promote wound closure and tissue repair in a dermal mouse model. Biomaterials. 166. 10.1016/j.biomaterials.2018.03.006.

The features of the present invention disclosed in the specification, the claims, and/or in the accompanying figures may, both separately and in any combination thereof, be material for realizing the invention in various forms thereof.

## Claims

1. A platelet-derived growth factor extract for use in a method of preventing or treating a tissue defect, wherein said platelet-derived growth factor extract is a heat-treated platelet-derived growth factor extract, preferably wherein, due to said heat-treatment of said platelet-derived growth factor extract, immune-activating molecules, preferably molecules of the complement system, have been inactivated.

2. The platelet-derived growth factor extract for use according to claim 1, wherein said tissue defect is a tissue inflammation, and/or is a tissue lesion deriving from a tissue injury and/or from a surgical procedure such as an organ transplantation, preferably wherein said tissue defect is a defect of a musculoskeletal tissue such as a tendon, a ligament, a cartilage, a muscle, or a bone, or is a defect of a cutaneous or subcutaneous tissue.

3. The platelet-derived growth factor extract for use according to claim 1 or 2, wherein said tissue defect is a disease selected from degenerative arthritis, a cartilage injury, a wound such as a dermal and transdermal wound, a muscle injury, and a tendinopathy such as a tendon injury or tendinitis.

4. The platelet-derived growth factor extract for use according to any of the foregoing claims, wherein said method of preventing or treating a tissue defect is a method of promoting wound healing.

5. The platelet-derived growth factor extract for use according to any of the foregoing claims, wherein said extract is prepared from blood of an animal, preferably a mammal, more preferably a horse, a dog, a cat, a cow, a pig, a sheep, a monkey, or a human.

6. The platelet-derived growth factor extract for use according to any of the foregoing claims, wherein said method of preventing or treating comprises administering said extract to a patient in need thereof, wherein said extract is prepared from autologous and/or allogeneic blood.

7. The platelet-derived growth factor extract for use according to any of the foregoing claims, wherein said extract is formulated as an allogeneic formulation.

8. The platelet-derived growth factor extract for use according to any of the foregoing claims, wherein, in said method, said extract is administered locally, topically, and/or systemically, preferably wherein, in said method, said extract is administered intratendinously, intramuscularly, intraligamentarily, intraosseously, intragastrointestinally, intraperitoneally, orally, nasally, mucosally, intrabronchially, intrapulmonarily, intradermally, intracoronarily, subcutaneously, intravenously, intravascularly, intrathecally, intraocularly, intraarticularly, or intranodally.

9. The platelet-derived growth factor extract for use according to any of the foregoing claims, wherein said heat-treatment comprises, preferably consists of, a heat-treatment of said extract at a temperature of from 50 °C to 70 °C for a period of 15 min to 60 min, preferably at a temperature of from 55 °C to 60 °C for 30 min to 60 min, more preferably at a temperature of 56 °C for 30 min.

10. The platelet-derived growth factor extract for use according to any of the foregoing claims, wherein said heat-treatment of said platelet-derived growth factor extract reduces immunogenic activity of said platelet-derived growth factor extract.

11. The platelet-derived growth factor extract for use according to any of the foregoing claims, wherein said extract comprises any of platelet-derived growth factor, transforming growth factor, platelet factor interleukin, platelet-derived angiogenesis factor, vascular endothelial growth factor, epidermal growth factor, insulin-like growth factor, and combinations of the foregoing.

12. The platelet-derived growth factor extract for use according to any of the foregoing claims, wherein, in said method, said extract is co-administered with a therapeutic agent.

13. The platelet-derived growth factor extract for use according to any of the foregoing claims, wherein, in said method, said extract is prepared by providing a blood sample comprising platelets and extracting growth factors from said platelets.

14. A pharmaceutical preparation comprising the platelet-derived growth factor extract as defined in any of claims 1-13, optionally further comprising any of fibronectin, collagen, hyaluronic acid, cord blood lysate, and an excipient.

15. A method of preparing a heat-treated platelet-derived growth factor extract as defined in any of claims 1-13, comprising the following steps:
a) providing a blood sample comprising platelets, and preparing a red blood cell fraction and a plasma fraction comprising platelets from said blood sample,
b) homogenizing said plasma fraction comprising platelets; optionally followed by obtaining a platelet-poor plasma supernatant and a platelet-rich pellet, and resuspending said platelet-rich pellet, preferably in a fraction of said platelet-poor plasma supernatant,
c) releasing cytokines and growth factors from said platelets in the preparation obtained in step b),
d) deactivating immune-activating molecules by heat-treatment of the growth factor extract obtained in step c), and
e) obtaining heat-treated platelet-derived growth factor extract, optionally comprising sterile filtration of said heat-treated platelet-derived growth factor extract,
preferably wherein said blood sample is an autologous blood sample and/or an allogeneic blood sample, and, optionally, said blood sample is a pooled blood sample.

16. The method according to claim 15, wherein said releasing comprises any of shock-freezing, treatment with CaCl2, ultrasound treatment of said platelets, preferably comprises shock-freezing followed by ultrasound treatment of said platelets, and/or wherein said deactivating comprises, preferably consists of, a heat-treatment at a temperature of from 50 °C to 70 °C for a period of 15 min to 60 min, preferably at a temperature of from 55 °C to 60 °C for 30 min to 60 min, more preferably at a temperature of 56 °C for 30 min.
